# EUROPEAN PATENT SPECIFICATION

(11) **EP 4 501 728 B1**
(45) Date of publication and mention of the grant of the patent: **25.02.2026**
(21) Application number: 24190910.0
(22) Date of filing: 25.07.2024
(51) Int. Cl.: B60W 30/14, B60W 50/12

(54) **EMERGENT DRIVING ASSISTANT BASED ON EMOTION AND GAZE**
EMERGENTER FAHRASSISTENT BASIEREND AUF EMOTION UND BLICK
ASSISTANT DE CONDUITE ÉMERGENTE BASÉ SUR L'ÉMOTION ET LE REGARD

(30) Priority: 31.07.2023 US 202363529807 P
(43) Date of publication of application: 05.02.2025
(73) Proprietor: Cerence Operating Company, Burlington, MA 01803 (US)
(72) Inventor: KANE, Mark, Burlington, MA 01803 (US); RUBIN, Brian, Burlington, MA 01803 (US); MONIRI, Mohammad Mehdi, 52070 Aachen (DE); FUNK, Markus, Burlington, MA 01803 (US); Kindermann, Daniel, 52070 Aachen (DE)
(74) Representative: Taruttis, Tilman

(56) References cited:
- CA-A1- 3 053 687
- US-A1- 2019 367 029
- US-A1- 2021 287 697
- US-A1- 2022 176 981
- US-A1- 2022 314 975

## Description

### Background

The invention relates to automotive electronics and in particular to electronic devices for assisting a driver in driving a vehicle.

Different segments of a road often have different speed limits. A driver may not be aware of when a speed limit has changed. As a result, a driver may inadvertently drive at a speed higher than that permitted.

It is useful to provide a warning to the driver who is driving in a manner that does not comply with the speed limit. Known methods of providing such warnings rely on the speed of the vehicle and the speed limit at the vehicle's location.

However, in some cases, the vehicle's driver may ignore such warnings. This often occurs when the driver is in an agitated state. When operating a vehicle in such a state, particularly in excess of permitted speeds, a heightened risk of accident arises.

CA 3053 687 A1 discloses a system configured to automatically reduce the speed of a truck upon entering a predefined geofenced area. The system overrides driver inputs that would otherwise cause speeding, for example by controlling fuel delivery or modifying acceleration control signals transmitted from the accelerator pedal to the electronic throttle controller and/or the engine control module (ECM). By employing geofencing technology to determine the vehicle's location and initiate speed reduction, the system enhances safety and ensures compliance with local speed regulations.

US 2022/314975 A1 relates to a drive assist system with an occupant monitoring apparatus, an information acquisition apparatus, a determination apparatus, and a drive assist apparatus. The drive assist apparatus includes one or more processors, which change a warning timing or a drive assist control intervention timing in accordance with the sleep state of a driver of a vehicle at least from a previous day, upon receiving information, from the determination apparatus, that the vehicle transitions from a stopped state to a traveling state. US 2011/169625 A1 relates to an apparatus for assisting safe operation of a vehicle. The apparatus includes an environment sensor system detecting hazards within the vehicle environment, a driver monitor providing driver awareness data, and an attention-evaluation module identifying hazards as sufficiently or insufficiently sensed by the driver by comparing the hazard data and the gaze track. An alert signal relating to the unperceived hazards can be provided. US 2021/287697 A1 relates to enhancing the accuracy of driver emotion recognition by distinguishing emotions triggered by media content from those arising from driving conditions. The system employs multiple sensors in combination with algorithms to analyse the driver's emotional state.

### Summary

The invention is as defined in the independent claims. Preferred embodiments of the invention are defined in the dependent claims.

In one aspect, the invention features a method that includes an emergent driving assistant emerging from an inactive state and relieving a driver of a vehicle from controlling a vehicle subsystem of the vehicle. The emergent driving assistant emerges from the inactive state upon determining that the vehicle is being operated in a manner that fails to comply with a constraint imposed on motion of vehicles on a section of a road and determining that the vehicle's driver exhibits a feature that is either a non-neutral emotional state or a non-neutral gaze state.

In some practices, the emergent driving assistant selects a vehicle subsystem and outputs a control signal that exercises at least some control over that subsystem.

Practices of the method include those in which the constraint is a speed limit and in which the subsystem is a speed-control subsystem. Among such practices are those in which the control signal curtails the driver's ability to drive faster than a particular speed but permits the driver to drive slower than that speed.

Among the practices of the method are those in which determining that a driver of the vehicle exhibits a non-neutral feature comprises making the determination based at least in part on a signal from a camera that is pointing at the driver, based at least in part on a signal from a microphone that is directed towards the driver, or based in part on both the signal from the camera and the signal from the microphone.

Still other practices include those in which determining the constraint is based at least in part on observations of signage by an external camera and those in which such a determination is based at least in part on a GPS signal.

Also among the practices of the invention are those in which the non-neutral feature is the non-neutral gaze state, those in which the feature is the non-neutral emotion, and those in which the feature that is present includes both the non-neutral emotion and the non-neutral gaze state.

Further practices include detecting that the non-neutral feature no longer exists and, consequently, restoring control over the vehicle subsystem.

In another aspect, the invention features an emergent driving assistant for a vehicle. The emergent driving assistant includes control circuitry that receives a constraint signal, which is indicative on a constraint on motion of a vehicle on a section of a road, a motion signal, which is indicative of the vehicle's motion, and a signal indicative of whether the driver exhibits either a non-neutral emotion or a non-neutral gaze state. The control circuitry is configured to output a control signal to a vehicle subsystem upon determining that: (1) the driver exhibits a non-neutral feature, which is either a non-neutral gaze state or a non-neutral emotion and (2) that the vehicle is being operated in a manner that violates the constraint. This control signal relieves the driver from being able to completely control that vehicle subsystem.

Embodiments include those in which the non-neutral feature comprises a non-neutral emotion and those in which the non-neutral feature comprises a non-neutral gaze state.

Still other embodiments include those in which the emergent driving assistant is configured to restore control over the vehicle subsystem upon determining that the non-neutral feature is no longer present.

As used herein, "gaze state" and "emotional state" are time-varying quantities that are observed during finite intervals. Thus, a "gaze state" can be classified as "neutral" or "non-neutral" based on variations in a gaze direction over time. Similarly, an "emotional state" can be classified as "neutral" or "non-neutral" based on variations in the driver's spectrum of emotions over time.

For example, if a gaze state indicates that the driver has been gazing in the same direction for an extended period, regardless of its direction, an inference can be made that emergence of the driver assistant may be required simply based on extended inactivity by the driver. Such a gaze, or "gaze state," could then be classified as "non-neutral" on that basis.

These and other features of the invention will be apparent from the following detailed description and the accompanying figures, in which:

### Description of Drawings

FIG. 1 shows a vehicle having an emergent driving assistant that provides assistance to a driver and
FIG. 2 shows a road to be traversed by the vehicle shown in FIG. 1, and
FIG. 3 shows details of a particular embodiment of the emergent driving assistant shown in FIG. 1.

### Detailed Description

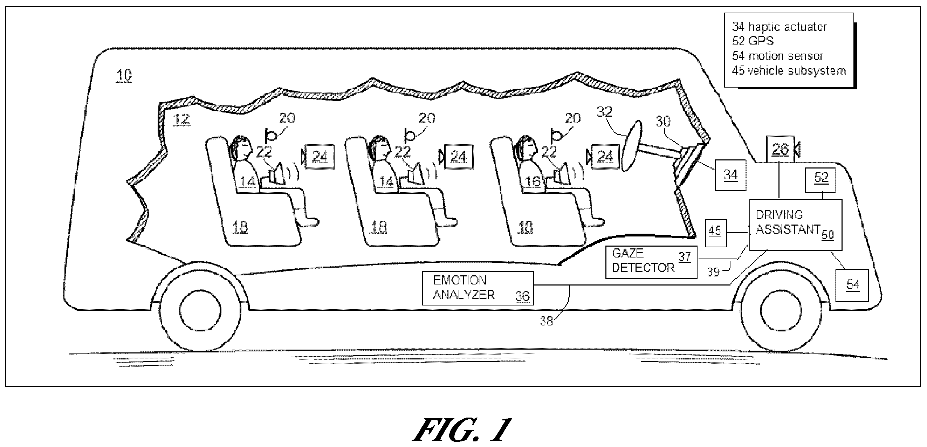

FIG. 1 shows a vehicle 10 having a passenger cabin 12 in which passengers 14 and a driver 16 sit in seats 18. Each seat 18 has an associated microphone 20, a loudspeaker 22, and an internal camera 24. The internal cameras 24 are directed towards various portions of the cabin 12. The vehicle 10 also includes external cameras 26. The external cameras 26 are directed towards the vehicle's environment.

The driver 16 faces an instrument panel 28 having a speedometer 30 integrated therein. In addition, the driver 16 has a steering wheel 32 for use in controlling the vehicle's direction. A haptic actuator 34 couples to one or more structures that, when vibrated or otherwise made to move, will attract the driver's attention. In the illustrated embodiment, the haptic actuator 34 couples to the steering wheel 32.

The vehicle 10 further includes an emotion analyzer 36 that receives inputs from the particular internal camera 24 and microphone 20 that are best situated to observe the driver 16. Based on features present in those inputs, the emotion analyzer 36 outputs an emotion signal 38. This emotion signal 38 indicates whether the driver's emotional state is in a neutral state or a non-neutral state. In some embodiments, the emotion signal 38 indicates an extent to which the driver's emotional state deviates from the neutral state.

In some embodiments, the emotion analyzer 36 also receives inputs from internal cameras 24 that face the passengers 14 and from microphones that are directed to the passengers 14. In such embodiments, the emotion signal 38 further includes information indicative of whether any one or more of the passengers 14 is a non-neutral emotional state. Such information is useful for assessing a driver's emotional state.

In the course of driving, a driver 16 spends much of the time gazing forward through the vehicle's windshield.

A driver 16 who periodically looks at the instrument panel 28, and in particular, the speedometer 30, is able to observe any visual warnings delivered via the instrument panel 28. Many warnings are delivered this way. Examples include a blue light indicating that high beams are on, a light for low fuel, a light indicating that the handbrake is engaged, a light indicating one's seat belt is not fastened, and various other lights whose meanings are often sufficiently obscure to require consulting the vehicle's manual. Such a driver would be considered to have a "neutral" gaze state.

As one drives, it is natural and indeed proper for one's gaze direction to shift away from the instrument panel 28. After all, it is hardly safe to drive a car while staring at the instrument panel 28 instead of at the road ahead. Thus, there may be extended periods during which the driver does not observe the instrument panel 28. During these periods, the driver will not be able to observe warnings delivered via the instrument panel 28. It is therefore useful to recognize the driver's gaze state as a function of time to identify extended periods during which the driver has not looked at the instrument panel so that warnings that might otherwise be missed can be delivered in a way that will allow them to be recognized. A driver 16 whose gaze includes extended periods of not looking at the instrument panel 28 would be considered to have a "non-neutral" gaze.

To promote the ability to observe the driver's gaze direction, and hence to determine the driver's gaze state, the vehicle 10 further includes a gaze detector 37 that receives inputs from the particular internal camera 24 and microphone 20 that are best situated to observe the driver 16. Based on features present in those inputs, the gaze detector 37 outputs a gaze signal 39. Embodiments include those in which the gaze signal 39 depends on head movement, eye movement, or a combination of both head and eye movement.

Based on this gaze signal 39, it is possible to determine the driver's gaze state based on a time-varying gaze direction. Armed with this information, it is possible to infer that a driver 16 may not notice a warning signal, either because the instrument panel 28 is not in the driver's field-of-view or because it is at the edge of the driver's field-of-view, where the eye's resolution is poorest.

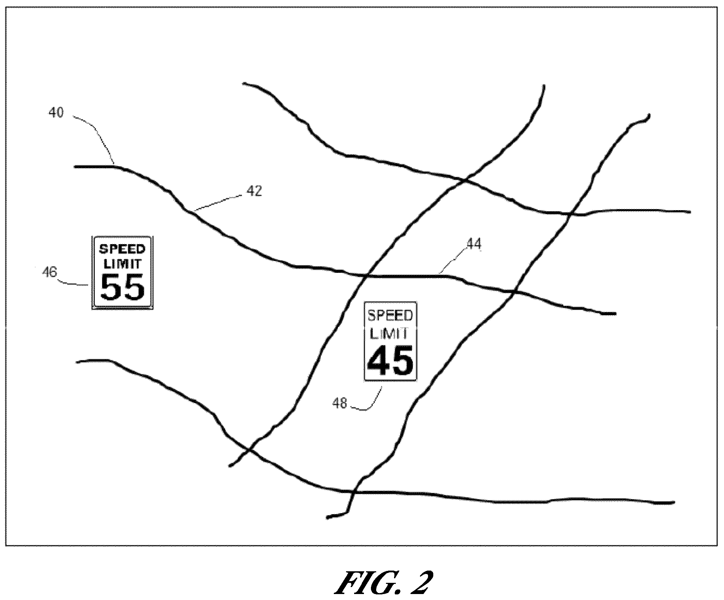

Referring to FIG. 2, a typical roadway 40 along which a vehicle 10 operates includes a first segment 42 and a second segment 44. A first constraint 46 imposes limits on the vehicle's motion within the first segment 42. Similarly, a second constraint 48 imposes limits on the vehicle's motion in the second segment 44.

A typical constraint 46, 48 is a maximum speed. However, certain roadways 40 also specify minimum speeds for vehicles 10 operating thereon. Thus, further examples of constraints 46, 48 include a minimum speed and a band of speeds between a minimum and maximum speed.

Still other examples of constraints 46, 48 include those on the vehicle's direction of travel. For example, a vehicle 10 proceeding in the wrong direction on a one-way street may be traveling below the speed limit but still not in compliance with a constraint 46, 48.

Yet other examples include a change in the vehicle's direction. For example, certain roadways 40, particularly in hilly areas, have "no passing" zones. In such cases, a constraint 46, 48 would permit changes in the vehicle's direction as needed to follow the roadway 40 but not changes that would result in changing lanes, e.g., weaving across lanes or departing from the lane on which the vehicle 10 is currently traveling.

It is preferable that the driver 16 operate the vehicle 10 in a manner that complies with the various constraints 46, 48. Referring back to FIG. 1, an emergent driving assistant 50 assists the driver 16 in operating within the constraint 46, 48 corresponding to the particular segment 42, 44. It does so by taking control over a vehicle subsystem 45 so as to curtail the driver's ability to operate the vehicle in a manner contrary to a constraint 46, 48. In some embodiments, the vehicle subsystem 45 is a speed control subsystem.

The driving assistant 50 relies on information concerning the applicable constraint 46, 48. In some embodiments, the driving assistant 50 uses input from the external camera 26 to identify signage from which it then extracts information concerning the relevant constraint 46, 48. In other embodiments, a GPS 52 maintains a library of constraints 46, 48 for specific locations and uses the vehicle's location to choose the relevant constraint 46, 48, which it then provides to the driving assistant 50.

The driving assistant 50 also relies on a motion sensor 54 that provides it with a motion signal 56. The motion signal 56 carries information concerning the vehicle's motion. In some embodiments, the motion signal 56 provides information on the vehicle's speed.

The driving assistant 50 is further configured to recognize circumstances that are indicative of the driver's state. In particular, the driving assistant 50 receives both the emotion signal 38 from the emotion analyzer 36 and the gaze signal 39 from the gaze detector 37. The emotion analyzer 36 classifies the driver 16 as being in a neutral emotional state or in a non-neutral emotional state. The gaze detector 37 classifies the driver 16 as having a neutral gaze state or a non-neutral gaze state.

To classify the driver 16 as being in one emotional state or the other, the emotion analyzer 36 observes various features of the driver 16. Each feature is assigned a numerical value and becomes an element of a measured feature vector. The emotion analyzer 36 then compares the measured feature vector with a standard feature vector. The standard feature vector is one whose elements have values that have been determined, for example through a machine learning process, to be associated with a driver 16 in the neutral state. A suitable comparison is that of setting a threshold value and classifying the driver 16 as being in a neutral state if the inner product of the measured feature vector and the standard feature vector exceeds that threshold and classifying the driver 16 as being in a non-neutral state otherwise.

Examples of features that are usable in both the measured and standard feature vectors are an extent to which the driver's face deviates from neutral expression, an extent to which the force of the driver's grip on the steering wheel deviate from a neutral force, an extent to which the amplitude and spectrum of the driver's voice indicates a departure from a neutral voice, an extent to which the driver 16 has failed to comply with applicable driving constraints over time, and prior history of the driver 16, including any history of moving violations.

The process carried out by the gaze detector 37 to classify a driver's gaze state is carried out in a similar way. The feature in this case is the time-varying gaze direction. This results in time series that is then compared with a time series associated with what has been defined to be a "neutral gaze state."

In some embodiments, a "neutral gaze state" occurs if the gaze duty cycle is above a neutral-gaze duty cycle. As used herein, a "gaze duty cycle" is obtained by dividing the time spent with a gaze directed towards the speedometer by the time spent with a gaze that is not directed towards the speedometer. If the gaze ratio is between zero and one-tenth, the driver is spending very little time looking at the speedometer. As a result, the gaze state is a non-neutral gaze state. If the gaze duty cycle is between unity and nine-tenths, the driver is staring too long at the speedometer. The gaze state is again a non-neutral gaze state.

In some embodiments, a "neutral emotional state" is defined relative to the set of feature vectors provided by training data. Each N-dimensional feature vector defines a point in a corresponding N-dimensional vector space. For the resulting distribution of points, there exists a closed surface *"S"* such that the fraction of points within S is α and the fraction outside S is 1-α, where α is a real number that is specified by the designer based on a desired error rate. A driver is said to be in a "neutral emotional state" if that driver's own N-dimensional feature vector is inside S. In some embodiments, α=0.8. In other embodiments, α=0.9. Embodiments in which a higher error rate is tolerable feature α=0.7 or lower.

In some embodiments, the driving assistant 50 relies on observed patterns in the driver's lack of compliance. In such embodiments, the driving assistant 50 observes a sequence of rapid lane changes or swerving indicates that the driver may also be impaired in some way. When combined with inferences made by the emotion analyzer 36 based on one or more internal cameras 24 and microphones 20, the driving assistant 50 is able infer whether the observed pattern has arisen from the driver's intoxication, from distraction by passengers 14, or from road rage. This, in turn, will inform whether or not to exercise control over a vehicle subsystem 45.

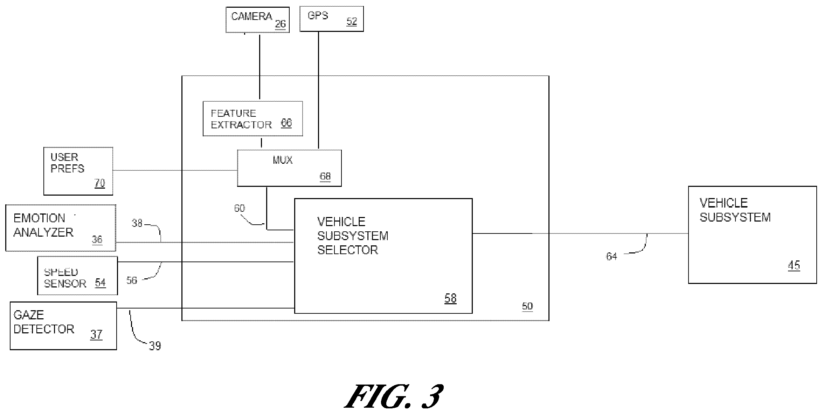

Referring now to FIG. 3, the driving assistant 50 features control circuitry 58 that receives the emotion signal 38, the motion signal 56, and the gaze signal 39 In addition, the control circuitry 58 receives a constraint signal 60. The constraint signal 60 indicates the constraint for that section of roadway that the vehicle 10 is driving on. Based on these inputs, the control circuitry 58 outputs a control signal 64 that relieves driver control over a particular vehicle subsystem 45 and takes over control of that subsystem.

In one example, the emotion signal 38 indicates a non-neutral emotional state and the motion signal 56 and constraint signal 60 together indicate that the driver is driving above a speed limit. In such cases, the driving assistant 50 relieves the driver 16 of complete control over the vehicle's speed control subsystem and substitutes a governor that limits the maximum speed. The driver 16 nevertheless continues to be able to vary the speed subject to the constraint on maximum speed. The driving assistant 50 thus curtails the driver's control only to the extent necessary to ensure compliance with the relevant constraint.

In another example, gaze signal 39 indicates a non-neutral gaze state and the motion signal 56 and constraint signal 60 together indicate that the driver is driving above a speed limit. In such cases, the driving assistant 50 carries out the same procedure, namely that of relieving the driver 16 of complete control over the vehicle's speed control subsystem and substituting a governor that limits the maximum speed. The driver 16 nevertheless continues to be able to vary the speed subject to the constraint on maximum speed. The driving assistant 50 thus curtails the driver's control only to the extent necessary to ensure compliance with the relevant constraint.

In the illustrated embodiment, there are two methods for obtaining the constraint signal 60. The first method comprises receiving the constraint 46, 48 directly from the GPS 52. The second method is to use a feature extractor 66 to receive a signal from the external camera 26 and to extract the relevant constraint 46, 48 from that signal.

Both the signal from the GPS 52 and the signal from the feature extractor 66 are provided to a multiplexer 68. The choice of which to use is made by stored user preferences 70 that are set by a user. In other embodiments, only one or the other method is available, in which case no multiplexer 68 is necessary.

The user preferences 70 also specify other operational details of the driving assistant 50. For example, by appropriately setting the user preferences 70, it is possible to disable the driving assistant 50 entirely or to disable the use of the emotion analyzer 36 by the driving assistant 50. Other settings include the amount of time non-compliant driving is tolerated before the driving assistant takes control of a vehicle subsystem 45. This is a useful feature to prevent jitter. Such jitter arises when the vehicle 10 is being driven at an average speed that is equal to the speed limit but with minor variances from that average.

## Claims

1. A method comprising an emergent driving assistant (50) emerging from an inactive state and relieving a driver (16) of a vehicle (10) from completely controlling a vehicle subsystem (45) of said vehicle (10), wherein emerging from said inactive state comprises
determining that said vehicle (10) is being operated in a manner that fails to comply with a constraint (46, 48) imposed on motion of vehicles on a section of a road (40) and
determining that said driver (16) exhibits a feature, said feature being selected from the group consisting of a non-neutral gaze state and a non-neutral emotional state.

2. The method of claim 1, further comprising selecting a vehicle subsystem (45) and outputting a control signal (64) that relieves said driver control over said vehicle subsystem (45).

3. The method of claim 1, wherein said constraint (46, 48) is a speed limit and wherein said vehicle subsystem (45) is a speed-control subsystem.

4. The method of claim 1, wherein outputting a control signal comprises outputting a control signal (64) that curtails said driver's ability to drive faster than a particular speed but permits said driver (16) to drive at a speed lower than said particular speed.

5. The method of claim 1, wherein determining that a driver (16) of said vehicle (10) exhibits said feature comprises making said determination based at least in part on a signal from a camera (24) that is pointing at said driver (16).

6. The method of claim 1, wherein determining that a driver (16) of said vehicle (10) exhibits said feature comprises making said determination based at least in part on a signal from a microphone (20) that is directed towards said driver (16).

7. The method of claim 1, further comprising determining said constraint (46, 48) based at least in part on observations of signage by an external camera (26) .

8. The method of claim 1, further comprising determining said constraint (46, 48) based at least in part on a GPS signal.

9. The method of claim 1, wherein said feature is said non-neutral emotional state.

10. The method of claim 1, wherein said feature is said non-neutral gaze state.

11. The method of claim 1, wherein said emergent driver assistant (50) carries out the steps of determining that said driver (16) no longer exhibits said feature, restoring said driver's control over said vehicle subsystem (45), and transitioning into its inactive state.

12. An apparatus comprising an emergent driving assistant (50) for a vehicle (10), said emergent driving assistant (50) comprising control circuitry (58),
wherein said control circuitry (58) receives a constraint signal (60), a motion signal (56), and a feature signal (38, 39),
wherein said feature signal (38, 39) is in one of a first state and a second state, said first state indicating that said driver (16) exhibits at least one of a non-neutral emotional state and a non-neutral gaze state and said second state indicating that said (16) driver exhibits both a neutral emotional state and a neutral gaze state,
wherein said constraint signal (60) is indicative of a constraint (46, 48) on motion of said vehicle (10) on a section of a road (40),
wherein said motion signal (56) is indicative of motion of said vehicle (10) on said section of said road (40),
wherein said control circuitry (58) is configured to determine, based on said constraint signal (60) and said motion signal (56), whether said vehicle (10) is being operated in a manner that violates said constraint (46, 48) on said motion of said vehicle (10) on said section of said (40) road, and
wherein said control circuitry (58) is configured to output a control signal (64) to a vehicle subsystem (45) when said feature signal (38, 39) is in said first state and when said vehicle (10) is being operated in a manner that violates said constraint (46, 48) on said motion of said vehicle (10) on said section of said road (40), said control signal (64) relieving said driver (10) from being able to completely control said vehicle subsystem (45).

## Patentansprüche

1. Verfahren, umfassend einen Notfallfahrassistenten (50), der aus einem inaktiven Zustand aktiviert wird und einem Fahrer (16) eines Fahrzeugs (10) das vollständige Steuern eines Fahrzeugsubsystems (45) des Fahrzeugs (10) abnimmt, wobei das Aktiviertwerden aus dem inaktiven Zustand umfasst
Bestimmen, dass das Fahrzeug (10) in einer Weise betrieben wird, die nicht mit einer Beschränkung (46, 48) übereinstimmt, die einer Bewegung von Fahrzeugen auf einem Abschnitt einer Straße (40) auferlegt ist, und
Bestimmen, dass der Fahrer (16) ein Merkmal aufweist, wobei das Merkmal aus der Gruppe ausgewählt ist, bestehend aus einem nicht-neutralen Blickzustand und einem nicht-neutralen Emotionszustand.

2. Verfahren nach Anspruch 1, ferner umfassend das Auswählen eines Fahrzeugsubsystems (45) und das Ausgeben eines Steuersignals (64), das dem Fahrer die Steuerung des Fahrzeugsubsystems (45) abnimmt.

3. Verfahren nach Anspruch 1, wobei die Beschränkung (46, 48) eine Geschwindigkeitsbegrenzung ist und wobei das Fahrzeugsubsystem (45) ein Geschwindigkeitsregelungssubsystem ist.

4. Verfahren nach Anspruch 1, wobei das Ausgeben eines Steuersignals das Ausgeben eines Steuersignals (64) umfasst, das die Fähigkeit des Fahrers einschränkt, schneller als mit einer bestimmten Geschwindigkeit zu fahren, aber dem Fahrer (16) erlaubt, mit einer Geschwindigkeit zu fahren, die niedriger ist als die bestimmte Geschwindigkeit.

5. Verfahren nach Anspruch 1, wobei das Bestimmen, dass ein Fahrer (16) des Fahrzeugs (10) das Merkmal aufweist, umfasst, die Bestimmung mindestens teilweise basierend auf einem Signal von einer Kamera (24), die auf den Fahrer (16) gerichtet ist, vorzunehmen.

6. Verfahren nach Anspruch 1, wobei das Bestimmen, dass ein Fahrer (16) des Fahrzeugs (10) das Merkmal aufweist, umfasst, die Bestimmung mindestens teilweise basierend auf einem Signal von einem Mikrofon (20), das auf den Fahrer (16) gerichtet ist, vorzunehmen.

7. Verfahren nach Anspruch 1, ferner umfassend das Bestimmen der Beschränkung (46, 48) mindestens teilweise basierend auf Beobachtungen von Beschilderung durch eine externe Kamera (26).

8. Verfahren nach Anspruch 1, ferner umfassend das Bestimmen der Beschränkung (46, 48) mindestens teilweise basierend auf einem GPS-Signal.

9. Verfahren nach Anspruch 1, wobei das Merkmal der nicht-neutrale Emotionszustand ist.

10. Verfahren nach Anspruch 1, wobei das Merkmal der nicht-neutrale Blickzustand ist.

11. Verfahren nach Anspruch 1, wobei der Notfallfahrerassistent (50) die Schritte des Bestimmens, dass der Fahrer (16) das Merkmal nicht mehr aufweist, des Wiederherstellens der Steuerung des Fahrers des Fahrzeugsubsystems (45) und des Übergehens in seinen inaktiven Zustand ausführt.

12. Einrichtung, umfassend einen Notfallfahrassistenten (50) für ein Fahrzeug (10), wobei der Notfallfahrassistent (50) eine Steuerschaltlogik (58) umfasst,
wobei die Steuerschaltlogik (58) ein Beschränkungssignal (60), ein Bewegungssignal (56) und ein Merkmalssignal (38, 39) empfängt,
wobei das Merkmalssignal (38, 39) in einem von einem ersten Zustand und einem zweiten Zustand ist, wobei der erste Zustand angibt, dass der Fahrer (16) mindestens einen von einem nicht-neutralen Emotionszustand und einem nicht-neutralen Blickzustand aufweist, und der zweite Zustand angibt, dass der Fahrer (16) sowohl einen neutralen Emotionszustand als auch einen neutralen Blickzustand aufweist,
wobei das Beschränkungssignal (60) eine Beschränkung (46, 48) einer Bewegung des Fahrzeugs (10) auf einem Abschnitt einer Straße (40) angibt,
wobei das Bewegungssignal (56) eine Bewegung des Fahrzeugs (10) auf dem Abschnitt der Straße (40) angibt,
wobei die Steuerschaltlogik (58) konfiguriert ist, um basierend auf dem Beschränkungssignal (60) und dem Bewegungssignal (56) zu bestimmen, ob das Fahrzeug (10) in einer Weise betrieben wird, die die Beschränkung (46, 48) der Bewegung des Fahrzeugs (10) auf dem Abschnitt der Straße (40) verletzt, und
wobei die Steuerschaltlogik (58) konfiguriert ist, um ein Steuersignal (64) an ein Fahrzeugsubsystem (45) auszugeben, wenn das Merkmalssignal (38, 39) in dem ersten Zustand ist und wenn das Fahrzeug (10) in einer Weise betrieben wird, die die Beschränkung (46, 48) der Bewegung des Fahrzeugs (10) auf dem Abschnitt der Straße (40) verletzt, wobei das Steuersignal (64) dem Fahrer (10) die Fähigkeit zum vollständigen Steuern des Fahrzeugsubsystems (45) abnimmt.

## Revendications

1. Procédé comprenant un assistant de conduite émergent (50) sortant d'un état inactif et soulageant un conducteur (16) d'un véhicule (10) de la commande complète d'un sous-système de véhicule (45) dudit véhicule (10), dans lequel émergeant dudit état inactif comprend
la détermination que ledit véhicule (10) est conduit d'une manière qui ne respecte pas une contrainte (46, 48) imposée au mouvement des véhicules sur une section d'une route (40) et
le fait de déterminer que ledit conducteur (16) présente une caractéristique, ladite caractéristique étant choisie dans le groupe constitué d'un état de regard non neutre et d'un état émotionnel non neutre.

2. Procédé selon la revendication 1, comprenant en outre la sélection d'un sous-système de véhicule (45) et l'émission d'un signal de commande (64) qui relève ladite commande du conducteur sur ledit sous-système de véhicule (45).

3. Procédé selon la revendication 1, dans lequel ladite contrainte (46, 48) est une limite de vitesse et dans lequel ledit sous-système de véhicule (45) est un sous-système de commande de vitesse.

4. Procédé selon la revendication 1, dans lequel l'émission d'un signal de commande comprend l'émission d'un signal de commande (64) qui réduit la capacité dudit conducteur à conduire plus vite qu'une vitesse particulière mais permet audit conducteur (16) de conduire à une vitesse inférieure à ladite vitesse particulière.

5. Procédé selon la revendication 1, dans lequel le fait de déterminer qu'un conducteur (16) dudit véhicule (10) présente ladite caractéristique comprend le fait de faire ladite détermination sur la base, au moins en partie, d'un signal provenant d'une caméra (24) qui pointe vers ledit conducteur (16).

6. Procédé selon la revendication 1, dans lequel le fait de déterminer qu'un conducteur (16) dudit véhicule (10) présente ladite caractéristique comprend la réalisation de ladite détermination sur la base, au moins en partie, d'un signal provenant d'un microphone (20) qui est dirigé vers ledit conducteur (16).

7. Procédé selon la revendication 1, comprenant en outre la détermination de ladite contrainte (46, 48) sur la base, au moins en partie, d'observations de la signalisation par une caméra externe (26).

8. Procédé selon la revendication 1, comprenant en outre la détermination de ladite contrainte (46, 48) sur la base, au moins en partie, d'un signal GPS.

9. Procédé selon la revendication 1, dans lequel ladite caractéristique est ledit état émotionnel non neutre.

10. Procédé selon la revendication 1, dans lequel ladite caractéristique est ledit état de regard non neutre.

11. Procédé selon la revendication 1, dans lequel ledit assistant de conduite émergent (50) effectue les étapes consistant à déterminer que ledit conducteur (16) ne présente plus ladite caractéristique, à restaurer la commande dudit conducteur sur ledit sous-système de véhicule (45), et à passer à son état inactif.

12. Appareil comprenant un assistant de conduite émergent (50) pour un véhicule (10), ledit assistant de conduite émergent (50) comprenant un ensemble de circuits de commande (58),
dans lequel ledit ensemble de circuits de commande (58) reçoivent un signal de contrainte (60), un signal de mouvement (56) et un signal caractéristique (38, 39),
dans lequel ledit signal caractéristique (38, 39) est dans l'un d'un premier état et d'un second état, ledit premier état indiquant que ledit conducteur (16) présente au moins l'un parmi un état émotionnel non neutre et un état de regard non neutre et ledit second état indiquant que ledit conducteur (16) présente à la fois un état émotionnel neutre et un état de regard neutre,
dans lequel ledit signal de contrainte (60) indique une contrainte (46, 48) sur un mouvement dudit véhicule (10) sur un tronçon d'une route (40),
dans lequel ledit signal de mouvement (56) indique un mouvement dudit véhicule (10) sur ladite section de ladite route (40),
dans lequel ledit ensemble de circuits de commande (58) sont configurés pour déterminer, sur la base dudit signal de contrainte (60) et dudit signal de mouvement (56), si ledit véhicule (10) est conduit d'une manière qui viole ladite contrainte (46, 48) sur ledit mouvement dudit véhicule (10) sur ladite section de ladite route (40), et
dans lequel ledit ensemble de circuits de commande (58) sont configurés pour délivrer en sortie un signal de commande (64) à un sous-système de véhicule (45) lorsque ledit signal caractéristique (38, 39) est dans ledit premier état et lorsque ledit véhicule (10) est conduit d'une manière qui viole ladite contrainte (46, 48) sur ledit mouvement dudit véhicule (10) sur ladite section de ladite route (40), ledit signal de commande (64) dispensant ledit conducteur (10) de la possibilité de commander complètement ledit sous-système de véhicule (45).
